# EUROPEAN PATENT APPLICATION

(11) **EP 1 938 855 A1**
(43) Date of publication of application: **02.07.2008**
(21) Application number: 06027037.8
(22) Date of filing: 29.12.2006
(51) Int. Cl.: A61M 16/04

(54) **Laryngeal mask airway without inflating**

(71) Applicant: Chang, Ti-Li, Taichung Hsien (TW)
(72) Inventor: Chang, Ti-Li, Taichung Hsien (TW)
(74) Representative: Hauck Patent- und Rechtsanwälte

(57) **Abstract**

A laryngeal mask airway has a dual-airway tube (10) and a laryngeal mask (20). The dual-airway tube (10) has a primary tube (11) and a secondary tube (12). The secondary tube (12) is formed parallel with the primary tube (11). The laryngeal mask (20) is attached detachable to or formed with the dual-airway tube (10) and has a top, a pointed front end, a rounded rear end, an opening (205) and a resilient seal (21). The resilient seal (21) is formed on the top of the laryngeal mask (20) and has a resilient web (211) and a seal (212). The resilient web (211) is formed on the top of the laryngeal mask (20) around the opening (205) and has a top edge. The seal (212) is formed on the top edge of the resilient web (211) and connects to the laryngeal mask (20) near the pointed front end.

## Description

### 1. Field of the Invention

The present invention relates to a laryngeal mask airway which could provide patients with spontaneous or positive-airway-pressure breathing, and more particularly to a laryngeal mask airway that can be used without inflating air into the laryngeal mask airway.

### 2. Description of Related Art

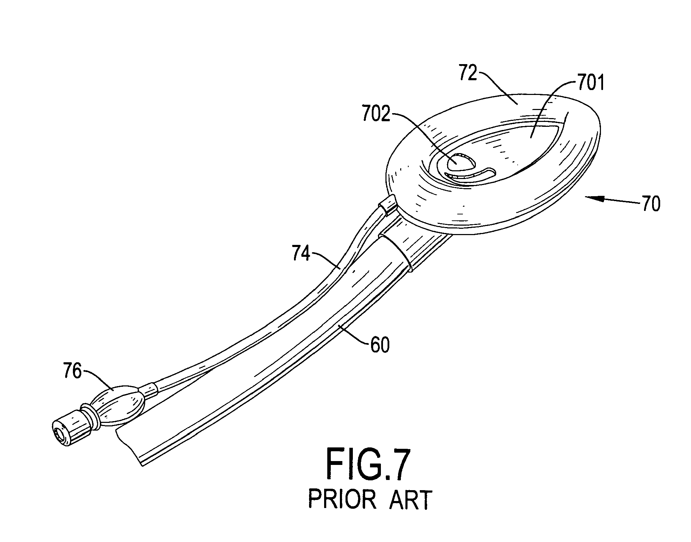

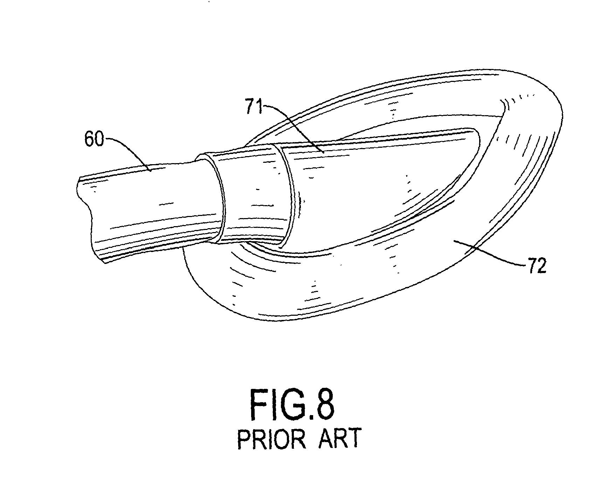

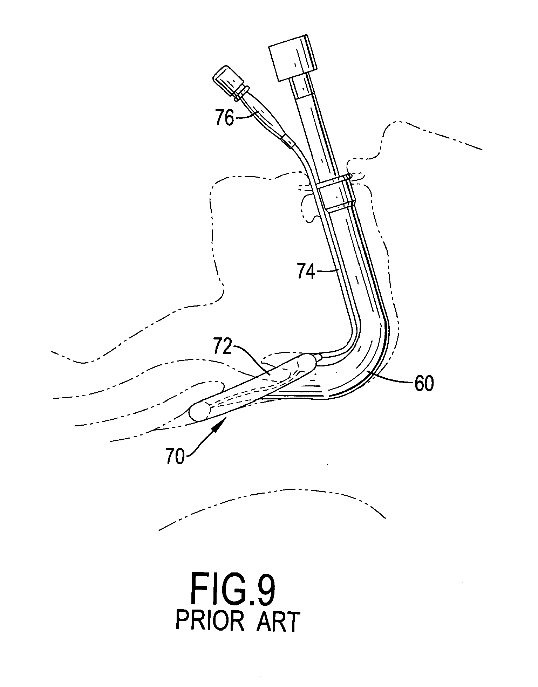

With reference to Figs. 7 to 9, a conventional laryngeal mask airway has an airway tube (60) and an inflated laryngeal mask (70). The airway tube (60) is a large-bore tube and has an inner end. The proximal end of the airway tube (60) is connected to the inflated laryngeal mask (70). The inflated laryngeal mask (70) is teardrop-shaped and has a curved base (71), a curved face (701), multiple vent holes (702), a bladder (72) and a drain tube (73). The curved base (71) may be triangular, is connected to the inner end of the airway tube (60) and has a bottom. The curved face (701) is defined in the bottom of the curve base (71). The vent holes (702) are defined in the curved face (701) to connect the inflated laryngeal mask (70) to the airway tube (60). The bladder (72) is inflatable, is formed around the curved base (71) and has a pointed front end, a rounded rear end, an inflation tube (74) and a pilot balloon (76). The inflation tube (74) is connected to the bladder (72) and has an inner end and an outer end. The inner end is connected to the rounded rear end of the bladder (72), and the outer end is a free end. The pilot balloon (76) is attached to the outer end of the inflation tube (74) with a valve. The drain tube (73) is connected to the airway tube (60) and communicates with the ventilation holes (702).

When the conventional laryngeal mask airway is used to allow a patient to breathe, the valve in the pilot balloon (76) deflates air from the bladder (72). Then, the inflated laryngeal mask (70) is carefully flattened against the hard palate and the soft palate, and the pointed front end of the bladder (72) is pushed into the esophagus until the ventilation holes (702) communicate with the larynxof the patient. With the bladder (72) around the openingof the larynx, a syringe connects to the valve in the pilot balloon (76) and injects air into the bladder (72) through the inflation tube (74) to inflate the bladder (72).

When the bladder (72) is inflated, no gap is formed between the bladder (72) and the larynx, and a medical gas can be introduced into the lungs of the patient. When pulling out the conventional laryngeal mask airway of the esophagus of patient, user needs to fully release the air in the bladder (72) through the valve in the pilot balloon (76).

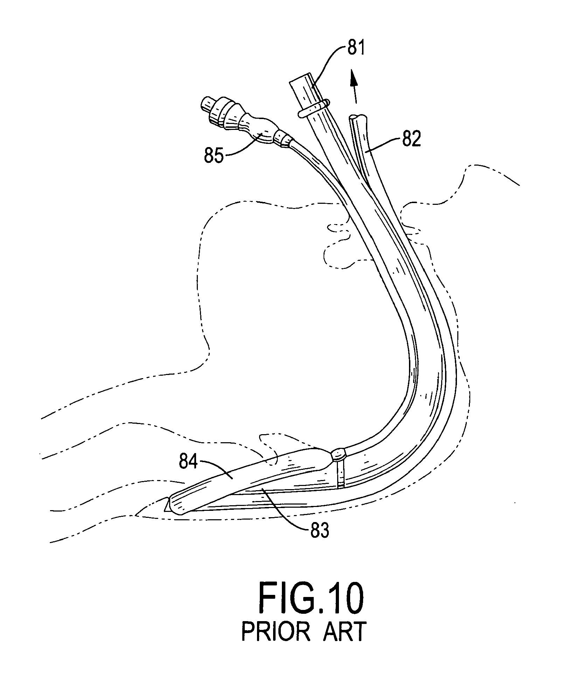

With further reference to Fig. 10, another conventional laryngeal mask airway has an airway tube (81) and a laryngeal mask (83). The airway tube (81) has a drain tube (82). The drain tube (82) is formed with the airway tube (81) in a two-barrel configuration. The laryngeal mask (83) is connected to the airway tube (81) and has a bladder (84) and an inflation tube (85). The inflation tube (85) is connected to the bladder (84).

However, conventional laryngeal mask airways have the following shortcomings.
1. The sealing pressure of the bladder (72, 84) of a conventional laryngeal mask airway covering the larynx and used to introduce medical gas into the lungs of a patient is high and will damage the mucous membranes of the larynx easily and cause ulceration of the larynx. If the pressure is reduced, the bladder (72, 84) does not seal the larynx well, and the medical gas escapes easily through gaps between the larynx and the bladder (72, 84).
2. The pointed front end of the bladder (72, 84) easily folds back on itself when the bladder (72, 84) is pressed into the esophagus. A folded bladder (72, 84) makes the patient uncomfortable, can cause reflex vomiting and will likely allow medical gas to escape.
3. When users use the conventional laryngeal mask airways, they need to purge air from the bladders (72, 84) before inserting the laryngeal masks (70, 83) into the larynx, and inject air into the bladders (72, 84) after inserting the laryngeal masks (70, 83) in the larynx. Furthermore, users have to exhaust the air in the bladders (72, 84) before retracting the bladder (72) from the esophagus of the patient. Furthermore, all the sealing function rely on the inflatable bladders (72, 84), and the broken or leak bladders (72, 84) would cause malfunction the device. Thus the conventional laryngeal mask airways are inconvenient and time-consuming in use.

The laryngeal mask airway in accordance with the present invention mitigates or obviates the aforementioned problems.

The main objective of the present invention is to provide a laryngeal mask airway that can be inserted quickly and easily without injecting air into the laryngeal mask airway.

The laryngeal mask airway in accordance with the present invention has a dual-airway tube and a laryngeal mask. The dual-airway tube has a lower end, a primary tube and a secondary tube. The secondary tube is formed parallel with the primary tube. The laryngeal mask is attached detachable to or formed with the lower end of the dual-airway tube and has a top, a pointed front end, a rounded rear end, an opening and a resilient seal. The resilient seal is formed on the top of the laryngeal mask and has a resilient web and a seal. The resilient web is formed on the top of the laryngeal mask around the opening and has a top edge. The seal is formed on the top edge of the resilient web and connects to the laryngeal mask near the front end.

Other objectives, advantages and novel features of the invention will become more apparent from the following detailed description when taken in conjunction with the accompanying drawings.

### IN THE DRAWINGS:

Fig. 1 is a perspective view of a laryngeal mask airway in accordance with the present invention;
Fig. 2 is an exploded perspective view of the laryngeal mask airway in Fig. 1;
Fig. 3 is a top view of the laryngeal mask airway in Fig. 1;
Fig. 4 is a cross sectional side view of the laryngeal mask airway along the line 4-4 in Fig. 3;
Fig. 5 is an optional side view of a laryngeal mask of the laryngeal mask airway in Fig. 1;
Fig. 6 is an operational side view of the laryngeal mask airway in Fig. 1 covering a patient's larynx;
Fig. 7 is a perspective view of a conventional laryngeal mask airway in accordance with the prior art;
Fig. 8 is an enlarged perspective view of the conventional laryngeal mask airway in Fig. 7;
Fig. 9 is an operational side view of the conventional laryngeal mask airway in Fig. 7 covering a patient's larynx; and
Fig. 10 is an operational side view of a second embodiment of a conventional laryngeal mask airway in accordance with the prior art covering a patient's larynx.

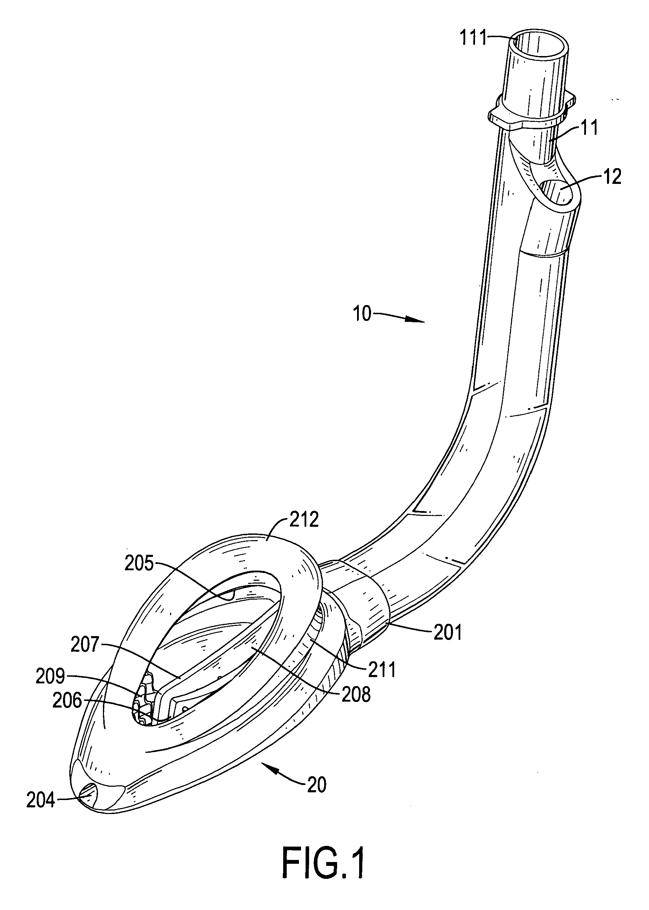

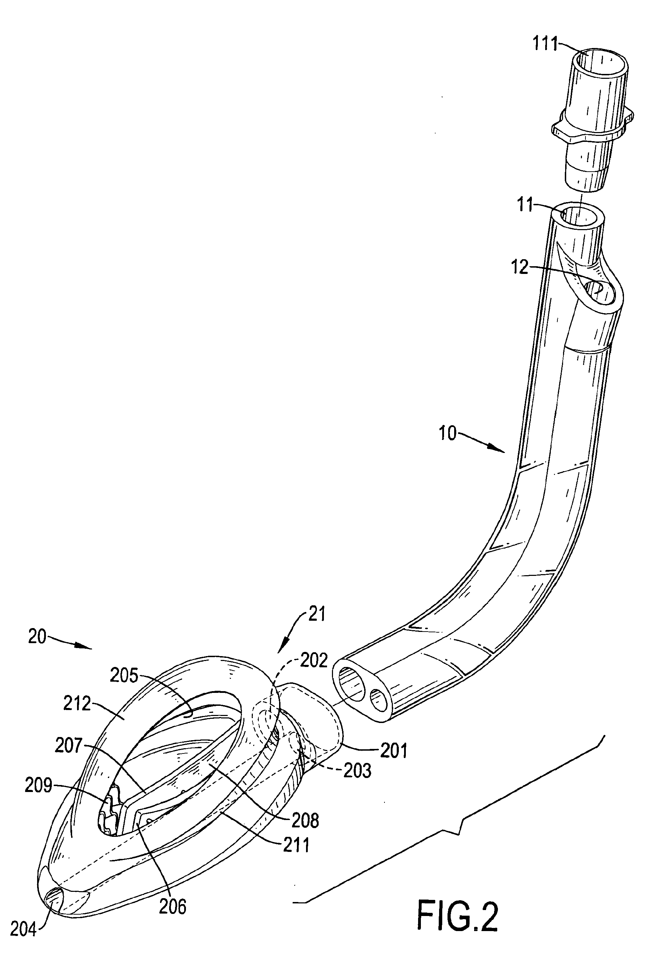

With reference to Figs. 1 and 2, a laryngeal mask airway in accordance with the present invention has a dual-airway tube (10) and a laryngeal mask (20).

The dual-airway tube (10) may be curved, may be plastic and has an upper end, a lower end, a primary tube (11), a secondary tube (12) and a relatively thick division wall. The primary tube (11) has an optional connector (111). The connector (111) is attached to the primary tube (11) near the upper end of the dual-airway tube (10). The secondary tube (12) is smaller than the primary tube (11) and is formed parallel with the primary tube (11). The division wall is formed between the primary tube (11) and the secondary tube (12) and keeps the primary tube (11), the secondary tube (12) or both from being blocked by a patient biting down on the dual-airway tube (10).

The laryngeal mask (20) may be elliptical, is attached to the lower end of the dual-airway tube (10), may be attached detachably to or formed with the lower end of the dual-airway tube (10), is resilient plastic and has a rounded rear end, a pointed front end, a top, a chamber, an optional joint (201), an optional aspirating inlet (204), an opening (205), an optional drain tube (206), at least one optional rib (207), an optional air passage (208), at least one optional elastic element (209) and a resilient seal (21).

The joint (201) is formed on the rounded rear end of the laryngeal mask (20), may be mounted detachably around the lower end of the dual-airway tube (10), may be formed on the lower end of the dual-airway tube (10) and has a ventilating hole (202) and an aspirating hole (203). The ventilating hole (202) is formed through the rounded rear end of the laryngeal mask (20) and connects the primary tube (11) of the dual-airway tube (10) to the chamber in the laryngeal mask (20). The aspirating hole (203) is formed through the rounded rear end of the laryngeal mask (20) adjacent to the ventilating hole (202) and communicates with the secondary tube (12) in the dual-airway tube (10).

The aspirating inlet (204) is formed through the pointed front end of the laryngeal mask (20).

The opening (205) is defined in the top of the laryngeal mask (20) and communicates with the chamber.

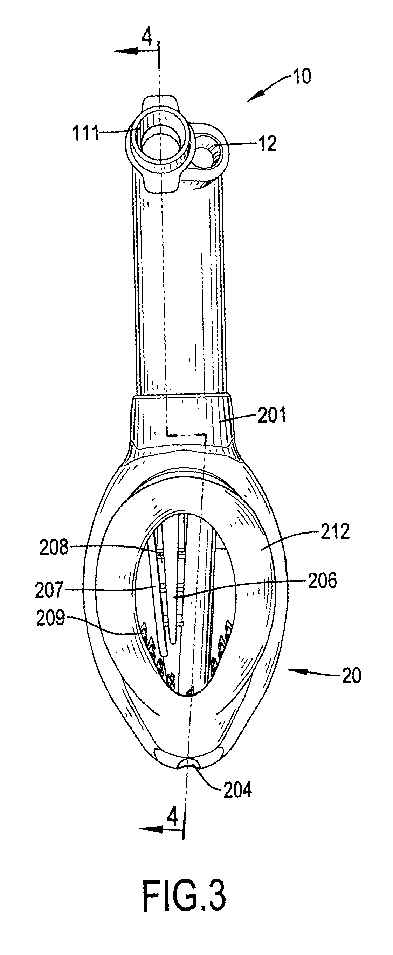

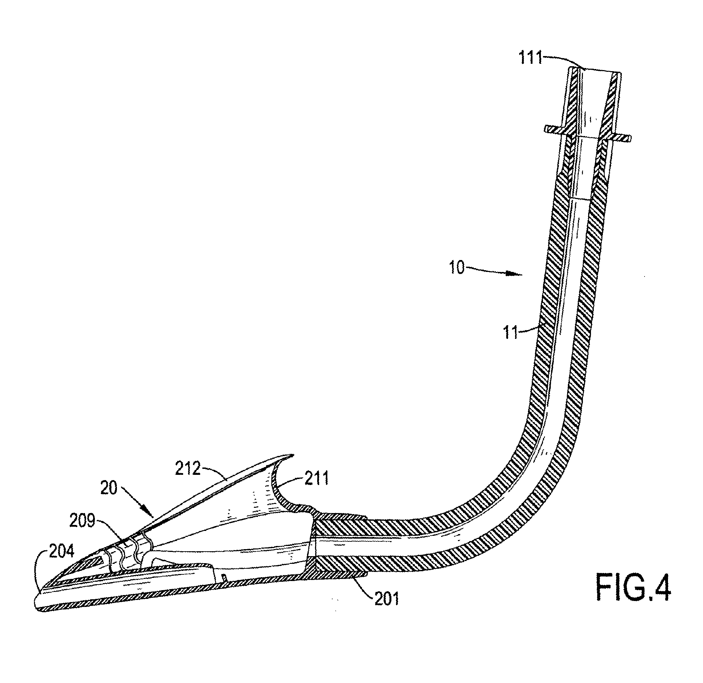

With further reference to Figs. 3 and 4, the drain tube (206) is formed in the chamber of the laryngeal mask (20) between the aspirating inlet (204) and the aspirating hole (203).

The at least one rib (207) is formed in the chamber in the laryngeal mask (20) near the ventilating hole (202).

The air passage (208) is defined between the drain tube (206) and the at least one rib (207) and communicates with the ventilating hole (202).

The at least one elastic element (209) is formed in the chamber in the laryngeal mask (20) beside the at least one rib (207).

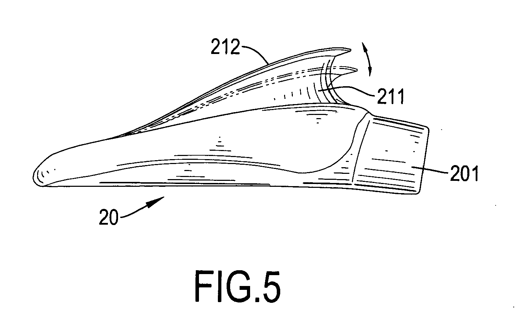

With further reference to Figs. 5, the resilient seal (21) is formed on and protrudes up from the top of the laryngeal mask (20) and has a resilient web (211) and a seal (212). The resilient web (211) may be C-shaped, is formed on the top of the laryngeal mask (20) around the opening (205), is tapered out from the pointed front end toward the round rear end and has a height and an upper edge. The height gradually increases from the pointed front end to the round rear end. The seal (212) is formed on and protrudes in and out from the upper edge of the resilient web (211) and is formed with the laryngeal mask (20) at the pointed front end.

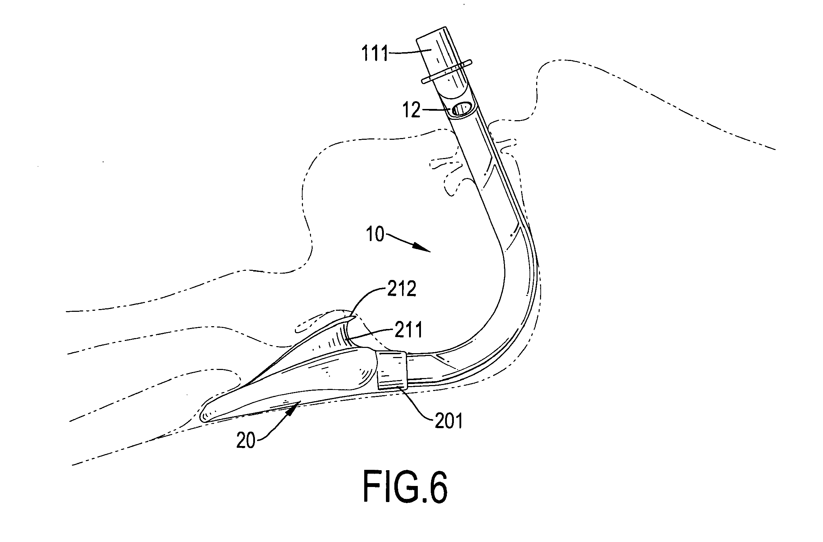

With reference to Fig. 6, the laryngeal mask airway is inserted into a patient throat to allow the patient to breathe by pushing the pointed front end of the laryngeal mask (20) into the esophagus until the opening (205) covers and communicates with the larynx and the aspirating inlet (204) communicates with the esophagus of the patient. The seal (212) is pressed around the larynx by the resilient web (211) and seals the larynx without having to inject air into the laryngeal mask (20).

When the seal (212) of the resilient seal (21) presses around the larynx of the patient, no gap is formed between the resilient web (211) and the larynx, and a medical gas can be injected into the lungs of the patient via the primary tube (11), the ventilating hole (202) and the opening (205). The at least one rib (207) keeps the epiglottis from obstructing the ventilating hole (202) in the joint (201). Furthermore, a fiber-scope can be inserted into the larynx through the primary tube (11), the ventilating hole (202), the air passage (208) and the opening (205).

The laryngeal mask airway as described has the following advantages.
1. When users use the laryngeal mask airway to allow a patient to breathe, they only need to gently push the pointed front end of the laryngeal mask (20) into the esophagus to cover and seal the larynx and do not need to inject air into the laryngeal mask (20). This completely obviates the necessity for conventional bladder (72, 84), keeps the over-pressure sealing of the laryngeal air mask from damaging the mucous membranes of the larynx and virtually eliminates ulceration of the larynx and problems with sealing the larynx well and medical gas escaping through gaps.
2. When the laryngeal mask (20) is pushed into the larynx, the seal (212) of the resilient seal (21) is pressed against the larynx by the resilient web (211) without having to inject air into the laryngeal mask (20), and no gap is formed between the resilient web (211) and the larynx. This can prevent the medical gas escaping from the resilient seal (21) of the laryngeal mask (20) when the bladders (72, 84) out of function due to the broken or leak happening.
3. The dual-airway tube (10) is composed of the primary tube (11) and the secondary tube (12), and the secondary tube (12) can aspirate the gastric juices or waste liquid the through the aspirating inlet (204), the drain tube (206), the aspirating hole and the secondary tube (12). Furthermore, the division wall of the dual-airway tube (10) keeps the primary tube (11) from blocking the medical gas when the patient bites the dual-airway tube (10).
4. The at least one rib (207) and the air passage (208) keep the epiglottis from obstructing the ventilating hole (202) of the joint (201). Then, the medical gas can flow freely into the lungs.

Even though numerous characteristics and advantages of the present invention have been set forth in the foregoing description, together with details of the structure and features of the invention, the disclosure is illustrative only. Changes may be made in the details, especially in matters of shape, size, and arrangement of parts within the principles of the invention to the full extent indicated by the broad general meaning of the terms in which the appended claims are expressed.

## Claims

1. A laryngeal mask airway **characterized in that**: the laryngeal mask airway comprising
a dual-airway tube (10) having
an upper end;
a lower end;
a primary tube (11);
a secondary tube (12) being formed parallel with the primary tube (11); and
a division wall formed between the primary tube (11) and the secondary tube (12); and
a laryngeal mask (20) being attached to the lower end of the dual-airway tube (10), being resilient plastic and having
a rounded rear end;
a pointed front end;
a top;
a chamber;
an opening (205) being defined in the top of the laryngeal mask (20) and communicating with the chamber; and
a resilient seal (21) being formed on and protruding up from the top of the laryngeal mask (20) and having
a resilient web (211) being formed on the top of the laryngeal mask (20) around the opening (205), being tapered out from the pointed front end toward the round rear end and having
a height gradually increasing from the pointed front end to the round rear end; and
an upper edge; and
a seal (212) being formed on and protruding in and out from the upper edge of the resilient web (211) and being formed with the laryngeal mask (20) at the pointed front end.

2. The laryngeal mask airway as claimed in claim 1, wherein the resilient web (211) is C-shaped.

3. The laryngeal mask airway as claimed in claim 1, wherein the laryngeal mask (20) is formed with the lower end of the dual-airway tube (10).

4. The laryngeal mask airway as claimed in claim 1, wherein the laryngeal mask (20) is attached detachable to the lower end of the dual-airway tube (10) and further has
a joint (201) being formed on the rounded rear end of the laryngeal mask (20), being mounted detachable around the lower end of the dual-airway tube (10) and having
a ventilating hole (202) being formed through the rounded rear end of the laryngeal mask (20) and connects the primary tube (11) of the dual-airway tube (10) to the chamber in the laryngeal mask (20); and
an aspirating hole (203) being formed through the rounded rear end of the laryngeal mask (20) adjacent to the ventilating hole (202) and communicating with the secondary tube (12) of the dual-airway tube (10);
an aspirating inlet (204) being formed through the pointed front end of the laryngeal mask (20); and
an drain tube (206) being formed in the chamber of the laryngeal mask (20) between the aspirating inlet (204) and the aspirating hole (203).

5. The laryngeal mask airway as claimed in claim 1, wherein the laryngeal mask (20) is elliptical.

6. The laryngeal mask airway as claimed in claim 3, wherein the laryngeal mask (20) further has
a joint (201) being formed on the rounded rear end of the laryngeal mask (20), being formed on the lower end of the dual-airway tube (10) and having
a ventilating hole (202) being formed through the rounded rear end of the laryngeal mask (20) and connecting the primary tube (11) of the dual-airway tube (10) to the chamber in the laryngeal mask (20); and
an aspirating hole (203) being formed through the rounded rear end of the laryngeal mask (20) adjacent to the ventilating hole (202) and communicating with the secondary tube (12) in the dual-airway tube (10);
an aspirating inlet (204) being formed through the pointed front end of the laryngeal mask (20); and
an drain tube (206) being formed in the chamber of the laryngeal mask (20) between the aspirating inlet (204) and the aspirating hole (203).

7. The laryngeal mask airway as claimed in claim 3, wherein the primary tube has a connector (111) being attached to the primary tube (11) near the upper end of the dual-airway tube (10).

8. The laryngeal mask airway as claimed in claim 4, wherein the laryngeal mask (20) has
at least one rib (207) being formed in the chamber in the laryngeal mask (20) near the ventilating hole (202);
an air passage (208) being defined between the drain tube (206) and the at least one rib (207) and communicating with the ventilating hole (202); and
at least one elastic element (209) being formed in the chamber in the laryngeal mask (20) beside the at least one rib (207).

9. The laryngeal mask airway as claimed in claim 6, wherein the laryngeal mask (20) has
at least one rib (207) being formed in the chamber in the laryngeal mask (20) near the ventilating hole (202);
an air passage (208) being defined between the drain tube (206) and the at least one rib (207) and communicating with the ventilating hole (202); and
at least one elastic element (209) being formed in the chamber in the laryngeal mask (20) beside the at least one rib (207).

## Amended claims

### Amended claims in accordance with Rule 137(2) EPC.

**1.** A laryngeal mask airway **characterized in that** the laryngeal mask airway comprises
a dual-airway tube (10) having
an upper end;
a lower end;
a primary tube (11);
a secondary tube (12) being formed parallel with the primary tube (11); and
a division wall formed between the primary tube (11) and the secondary tube (12); and
a laryngeal mask (20) being formed as a single hollow piece, being attached to the lower end of the dual-airway tube (10), being resilient plastic and having
a rounded rear end;
a pointed front end;
a top;
a chamber;
an opening (205) being defined in the top of the laryngeal mask (20) and communicating with the chamber; and
a resilient seal (21) being formed on and protruding up from the top of the laryngeal mask (20) and having
a resilient web (211) being formed on the top of the laryngeal mask (20) around the opening (205), being tapered out from the pointed front end toward the round rear end and having
a height gradually increasing from the pointed front end to the round rear end; and
an upper edge; and
a seal (212) being formed on and protruding in and out from the upper edge of the resilient web (211) and being formed with the laryngeal mask (20) at the pointed front end.

**2.** The laryngeal mask airway as claimed in claim 1, wherein the resilient web (211) is C-shaped.

**3.** The laryngeal mask airway as claimed in claim 1, wherein the laryngeal mask (20) is formed with the lower end of the dual-airway tube (10).

**4.** The laryngeal mask airway as claimed in claim 1, wherein the laryngeal mask (20) is attached detachable to the lower end of the dual-airway tube (10) and further has
a joint (201) being formed on the rounded rear end of the laryngeal mask (20), being mounted detachable around the lower end of the dual-airway tube (10) and having
a ventilating hole (202) being formed through the rounded rear end of the laryngeal mask (20) and connects the primary tube (11) of the dual-airway tube (10) to the chamber in the laryngeal mask (20); and
an aspirating hole (203) being formed through the rounded rear end of the laryngeal mask (20) adjacent to the ventilating hole (202) and communicating with the secondary tube (12) of the dual-airway tube (10);
an aspirating inlet (204) being formed through the pointed front end of the laryngeal mask (20); and
an drain tube (206) being formed in the chamber of the laryngeal mask (20) between the aspirating inlet (204) and the aspirating hole (203).

**5.** The laryngeal mask airway as claimed in claim 1, wherein the laryngeal mask (20) is elliptical.

**6.** The laryngeal mask airway as claimed in claim 3, wherein the laryngeal mask (20) further has
a joint (201) being formed on the rounded rear end of the laryngeal mask (20), being formed on the lower end of the dual-airway tube (10) and having
a ventilating hole (202) being formed through the rounded rear end of the laryngeal mask (20) and connecting the primary tube (11) of the dual-airway tube (10) to the chamber in the laryngeal mask (20); and
an aspirating hole (203) being formed through the rounded rear end of the laryngeal mask (20) adjacent to the ventilating hole (202) and communicating with the secondary tube (12) in the dual-airway tube (10);
an aspirating inlet (204) being formed through the pointed front end of the laryngeal mask (20); and
an drain tube (206) being formed in the chamber of the laryngeal mask (20) between the aspirating inlet (204) and the aspirating hole (203).

**7.** The laryngeal mask airway as claimed in claim 3, wherein the primary tube has a connector (111) being attached to the primary tube (11) near the upper end of the dual-airway tube (10).

**8.** The laryngeal mask airway as claimed in claim 4, wherein the laryngeal mask (20) has at least one rib (207) being formed in the chamber in the laryngeal mask (20) near the ventilating hole (202);
an air passage (208) being defined between the drain tube (206) and the at least one rib (207) and communicating with the ventilating hole (202); and
at least one elastic element (209) being formed in the chamber in the laryngeal mask (20) beside the at least one rib (207).

**9.** The laryngeal mask airway as claimed in claim 6, wherein the laryngeal mask (20) has at least one rib (207) being formed in the chamber in the laryngeal mask (20) near the ventilating hole (202);
an air passage (208) being defined between the drain tube (206) and the at least one rib (207) and communicating with the ventilating hole (202); and
at least one elastic element (209) being formed in the chamber in the laryngeal mask (20) beside the at least one rib (207).
